# EUROPÄISCHE PATENTSCHRIFT

(11) **EP 1 871 733 B1**
(45) Veröffentlichungstag und Bekanntmachung des Hinweises auf die Patenterteilung: **28.11.2012**
(21) Anmeldenummer: 06724056.4
(22) Anmeldetag: 05.04.2006
(51) Int. Cl.: C07C 37/74, C07C 37/88, C07C 39/07, C07C 37/00, C07C 39/06

(54) **VERFAHREN ZUR HERSTELLUNG VON CARDANOL (II)**
METHOD FOR PRODUCING CARDANOL (II)
PROCEDE POUR PREPARER DU CARDANOL (II)

(30) Priorität: 14.04.2005 DE 102005017125
(43) Veröffentlichungstag der Anmeldung: 02.01.2008
(73) Patentinhaber: Cognis IP Management GmbH, 40589 Düsseldorf (DE)
(72) Erfinder: SATO, Setsuo, 12328-190-Jacarei - SP (BR); BUENO DE ALMEIDA, Wanderson, 12246-300 Sao Jose dos Campos - SP (BR); BUENO, Ramiro, Carielo, 12328-390 Jacarei - SP (BR); ARAUJO, Alexssander Shigueru, 12241-070Sao José d os Campos - SP (BR)
(86) Internationale Anmeldenummer: PCT/EP2006/003108
(87) Internationale Veröffentlichungsnummer: WO 2006/108546

(56) Entgegenhaltungen:
- GB-A- 2 066 820
- GB-A- 2 262 525
- US-A- 4 352 944

## Beschreibung

### Gebiet der Erfindung

Die Erfindung betrifft ein verbessertes Verfahren zur Herstellung von Cardanol aus CNSL. Das Verfahren zeichnet sich dadurch aus, daß das so erhältliche Cardanol weniger Nebenprodukte aufweist, als das übliche kommerziell erhältliche Cardanol.

### Stand der Technik

Phenalkamine sind eine noch relativ junge Klasse von Epoxidharzhärtern. Es handelt sich dabei um Umsetzungsprodukte (Kondensationsprodukte) von Cardanol (I) , das chemisch ein C₁₅-Alkylphenol darstellt und ein Hauptbestandteil des aus den Schalen von Cashew-Nüssen zugänglichen Öls (des sogenannten CNSL = cashew nut shell liquid) ist, mit aliphatischen (primären bzw. sekundären ) Aminen und Formaldehyd. 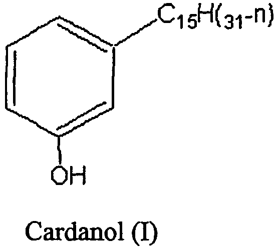 Bezüglich der Klasse der Phenalkamine sei auf folgende Publikation verwiesen: Zhishen Dai et al., "Phenalkamines: Multipurpose Epoxy Curing Agent"; Cardolite Corporation, Newark, New Jersey, USA; Reprint SPI-ERF Conference, September 1994.

Bekanntlich enthält rohes CNSL überwiegend eine Verbindung, die als Anacardic acid (II) bezeichnet wird. Bei der Destillation von CNSL in Gegenwart von Säure erhält man eine Zusammensetzung, die überwiegend Cardanol enthält, als Nebenprodukt jedoch Cardol (III). Vergleiche hierzu zum Beispiel US-B-6,262,148 und US-B-6,229,054. Dies steht im Einklang mit Untersuchungen der Anmelderin, wonach bei der Destillation von rohem CNSL eine Zusammensetzung erhalten wird, die überwiegend Cardanol, als Nebenprodukt jedoch Cardol sowie untergeordnete Mengen an 2-Methylcardol und Anacardic acid enthält.

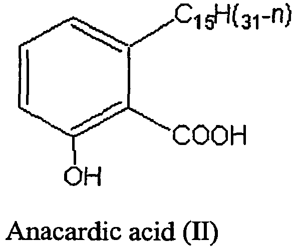

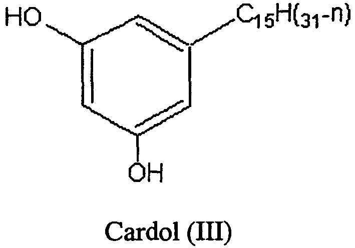

Das auf diese Weise erhaltene Cardanol/Cardol-Gemisch hat im wesentlichen drei technische Nachteile:
- Bei seiner Herstellung durch Destillation aus dem rohen CNSL treten Wertverluste auf, da bei diesem Vorgang ein Teil des Cardanols durch Polymerisation verlorengeht, so daß letztendlich die Cardanol-Ausbeute im Destillat nur 50-60% beträgt.
- Das zunächst leicht gelblich gefärbte Cardanol/Cardol-Gemisch verändert sich bei Lagerung: es kommt rasch zu einer Braunfärbung. Diese unerwünschten Farbveränderungen werden auf die Anwesenheit von Cardol und anderen unbekannten Komponenten, die bei der Gewinnung von CNSL oder der Destillation von CNSL bei hohen Temperaturen gebildet werden, zurückgeführt.
- Auch Folgeprodukte des Cardanol/Cardol-Gemisches zeigen bei Lagerung die geschilderten unerwünschten Farbveränderungen.

Es ist vorgeschlagen worden, die Farbstabilität von Cardanol/Cardol-Gemischen dadurch zu verbessern, indem man den Gehalt an Cardol durch ganz spezielle Maßnahmenvermindert. Hierzu ist vorgeschlagen worden, zunächst das im CNSL vorhandene Cardol weitgehend selektiv mit Aldehyden, Aminen bzw. Basen und Hydroxiden von Alkali- und Erdalkalimetallen umzusetzen, und anschließend das nicht umgesetzte Cardanol abzudestillieren. Im Hinblick auf diese Verfahren zur Gewinnung von Cardanol mit verbesserter Farbstabilität sei auf die Dokumente GB-A-2,152,925**,** GB-A-2,066,820 und US-A-4,352,944 verwiesen.

### Beschreibung der Erfindung

Die Aufgabe der vorliegenden Erfindung bestand darin, ein Verfahren zur Herstellung von farbstabilem Cardanol bereitzustellen. Darüber hinaus sollten auch Folgeprodukte des nach diesem Verfahren hergestellten Cardanols, insbesondere Phenalkamine, farbstabil sein.

Überraschenderweise wurde gefunden, daß dies gelingt, indem folgende Prozedur durchgeführt wird: (a) rohes CNSL einer raschen Destillation unterworfen wird, wobei Temperatur und/oder Verweilzeit möglichst gering gehalten werden, um unerwünschte Reaktionen wie Oxidation auf ein möglichst geringes Maß zu beschränken, (b) das Destillat einer speziellen chemischen Behandlung nebst (c) anschließender fraktionierender Destillation unterworfen wird. Vorzugsweise kann dann zusätzlich eine weitere Behandlung des Destillats mit chemischen Adsorbentien erfolgen.

Die essentiellen Verfahrensschritte des erfindungsgemäßen Verfahrens sind wie folgt:
1. Destillation, insbesondere Kurzwegdestillation, von rohem CNSL
2. Umsetzung des Destillats ("Roh-Cardanol") mit Essigsäureanhydrid
3. Fraktionierende Destillation der Reaktionsmischung von Schritt 2.

Der Hauptlauf von Schritt 3 kann gewünschtenfalls (optional) mit geringen Mengen Adsorbentien und/oder Reduktionsmitteln nachbehandelt werden. Dies ist eine optionale Maßnahme.

Gewünschtenfalls kann bereits das rohe CNSL mit Essigsäureanydrid vorbehandelt werden (vor Schritt 1).

Gegenstand der Erfindung ist ein Verfahren zur Herstellung einer farbstabilem Zusammensetzung enthaltend Cardanol, wobei man
1. rohes CNSL (cashew nut shell liquid) zunächst einer Destillation, insbesondere einer Kurzwegdestillation, unterwirft,
2. das dabei erhaltene Destillat mit Essigsäureanhydrid umsetzt und
3. anschließend eine fraktionierende Destillation durchführt.

Das durch das erfindungsgemäße Verfahren zugängliche Produkt enthält mindestens 98% Cardanol-Isomere. Cardol und Methylcardole sind in nur geringen Mengen enthalten, vorzugsweise unterhalb von 0,05%.

Das im Zuge des erfindungsgemäßen Verfahrens eingesetzte CNSL ist natürlichen Ursprungs - es wird wie bereits gesagt aus den äußeren Schalen von Cashewnüssen (Nüsse des Baumes Anacardium Occidentale) durch Extraktion gewonnen und kann hinsichtlich seiner Zusammensetzung variieren. Typischerweise enthält es 60-65% Cardanol, 2-10% Cardol, 10-15% Oligomere/Polymere und 0-2% 2-Methylcardanol und Anacardic acid.

Durch das erfindungsgemäße Verfahren wird eine farbstabile Zusammensetzung enthaltend Cardanol erhalten. Diese Zusammensetzung ist nicht nur als solche bei Lagerung farbstabil, sondern die auf Basis dieser Zusammensetzung hergestellten Phenalkamine sind es ebenfalls. Außerdem zeichnet sich die Zusammensetzung dadurch aus, dass sie im Vergleich zu den bisherigen handelsüblichen Produkten eine bessere Hautverträglichkeit aufweist, wodurch Handhabung und Transport sicherer werden.

In einer bevorzugten Ausführungsform wird der Hauptlauf von Schritt 3 des erfindungsgemäßen Verfahrens mit geringen Mengen Adsorbentien und/oder Reduktionsmitteln nachbehandelt. Durch den Einsatz von Adsorbentien und/oder Reduktionsmitteln wird eine weitere Erhöhung der Farbstabilität erzielt.

Zu den oben genannten drei obligatorischen Schritten des erfindungsgemäßen Verfahrens sei folgendes angemerkt:
- **Schritt 1** beinhaltet eine weitgehende Abtrennung von Oligomeren (mit Molgewichten im Bereich von 1000 bis 3000) im rohen CNSL. Die Abtrennung geschieht durch Destillation, wobei bereits eine einfache Kurzwegdestillation zu ausgezeichneten Ergebnissen führt. Bei dieser Abtrennung werden in der Regel etwa 15-20 Gew.-% des CNSL in Form der genannten Oligomeren entfernt. Die Kurzwegdestillation ist als Destillationsmethode bevorzugt. Sie ist mit einer raschen Prozessführung verbunden, wodurch Nebenprozesse wie Polymerisation oder Oxidation so weit als möglich unterbunden bzw. minimiert werden. Vorzugsweise wird die Kurzwegdestillation bei Temperaturen im Bereich von 220 bis 260 °C und Drucken im Bereich von 1 bis 5 mmHg durchgeführt. Vorzugsweise setzt man eine Kurzweg-Destillationsapparatur ein, die mit einem Vorverdampfer ausgerüstet ist. Der Hauptlauf, das eigentliche Destillat aus Schritt 1, wird nachfolgend auch als Roh-Cardanol bezeichnet.
- In **Schritt 2** wird das Destillat von Schritt 1 mit Essigsäureanhydrid umgesetzt. Dabei werden unerwünschte Farbträger chemisch gebunden. Die Menge an Essigsäureanhydrid beträgt vorzugsweise 1 bis 5 Gew.% - bezogen auf das aus Schritt 1 stammende Destillat. Eine Menge von etwa 2 bis 3 Gew.-% ist dabei besonders bevorzugt. Die Reaktionstemperatur wird vorzugsweise auf Werte im Bereich von 50 bis 70 °C eingestellt. Die Reaktionszeit wählt man vorzugsweise im Bereich von 30 bis 90 Minuten, insbesondere etwa 1 Stunde. Gebildete Essigsäure wird vorzugsweise kontinuierlich aus dem System entfernt (stripping off).
- Anschließend wird das Gemisch aus **Schritt 3** einer fraktionierenden Destillation unterworfen. Vorzugsweise setzt man dabei eine Fraktionierkolonne mit mehr als 6 theoretischen Böden ein, wobei die Destillation kontinuierlich durchgeführt wird. In einer Ausführungsform setzt man zur kontinuierlichen fraktionierenden Destillation eine Fraktionierkolonne üblichen Typs mit Kopf, Verstärkungsteil, Zulaufboden, Abtriebsteil und Sumpf ein. Dabei werden die Cardanole am oberen Ende (Kopf) der Kolonne entnommen, die Cardole am unteren Ende (Sumpf). Um ein Überhitzen zu vermeiden, arbeitet man vorzugsweise bei möglichst niederen Temperaturen. Bevorzugte Bedingungen sind: 180 bis 210 °C / 0,5 bis 1,5 mmHg am Kopf der Fraktionierkolonne und 230 bis 260 °C / 1,5 bis 3 mm HG am unteren Ende der Kolonne. Der Produktstrom am unteren Ende der Kolonne enthält eine Fraktion, die reich an Cardolen ist und nur geringe Anteile an Cardanolen und Essigsäureestem aufweist.

Während die Schritte 1 bis 3 für das erfindungsgemäße Verfahren obligatorisch sind, ist **Schritt 4** optional. Hierbei werden noch vorhandene Verunreinigungen weitgehend entfernt. An sich besteht dabei keinerlei Beschränkungen hinsichtlich der Natur der eingesetzten Adsorbentien bzw. Reduktionsmittel. Beispiele geeigneter Reduktionsmittel sind etwa Nariumhydrosulfit (Na₂S₂O₄), Natriummetabisulfit (Na₂S₂O₅), Natriumborhydrid (NaBH₄), Lithiumaluminumhydrid (LiAlH₄), Zinnchlorid (SnC12), Magnesium Silicate. Als Adsorbentien kommen beispielsweise Magnesiumsilikat oder chemisch äquivalente Verbindungen zum Einsatz. Die Menge der Adsorbentien bzw. Reduktionsmittel kann gering gehalten werden. Vorzugsweise setzt man Mengen von 0,1 bis 5 Gew.-% (bezogen auf den in Schritt 3 erhaltenen Hauptlauf) ein, wobei Mengen um etwa 1 Gew.-% besonders bevorzugt sind.

Ein weiterer Erfindungsgegenstand ist die Verwendung Cardanol-haliger Mischungen, erhältlich nach dem erfindungsgemäßen Verfahren, zur Herstellung farbstabiler Phenalkamine.

### Beispiele

### Beispiel 1: Erfindungsgemäßes Verfahren

Schritt 1: 1250 g/h CNSL (von Firma Resibras) wurden kontinuierlich in eine Kurzweg-Destillationsapparatur eingespeist, die mit einem Vorverdampfer ausgestattet war, wobei bei 240 °C / 1 mmHg bzw. 170 °C / 5 mmHg gefahren wurde. Bedingungen: Vorlauf im Vorverdampfer = 63 g/h; Hauptlauf = 940 g/h.
Schritt 2: 5000 g des Destillates aus dem Hauptlauf von Schritt 1 ("rohes Cardanol") wurden 1 Stunde lang bei 60 °C mit 12,5 g Essigsäureanhydrid umgesetzt.
Schritt 3: Das Material aus Schritt 2 wurde kontinuierlich in einen Entgaser (170 °C / 5 mmHg) eingespeist, um die in Schritt 2 gebildete Essigsäure zu entfernen. Vom Entgaser wurde das Material in eine Fraktionierkolonne geleitet, die kontinuierlich betrieben wurde (oben: 200 °C / 1 mmHg; unten: 250 °C / 3 mmHg; Rückflußverhältnis: 0,30). 80% des eingesetzten Materials wurden oben entnommen (Cardanol-reiche Fraktion), 20% unten (Cardol-reiche Fraktion mit geringen Anteilen von Cardanol und Essigsäureestem).
Step 4: Das Kopfprodukt von Schritt 3 wurden anschließend mit 7 g Magnesiumsilikat gemischt und die Mischung 30 Minuten bei 50 °C gerührt, anschließend wurde unter Einsatz eines Sparkler-Filters filtriert. Auf diese Weise wurde ein reines und farbstabiles Cardanol erhalten.

### Beispiel 2: Bestimmung der Farbstabilität

Die Farbe (Gardner-Fabrwerte) des gemäß Beispiel 1 erhaltenen Produktes wurden sofort gemessen. Anschließend wurde das Produkt bei 90 °C in einem elektrisch beheizten Ofen gelagert und die Farbe nach 1 Tag und nach 2 Tagen bestimmt. Ebenso wurde ein handelsübliches Cardanol dem gleichen Lagerungstest unterworfen und die Farbwerte in gleicher Weise bestimmt. Die Ergebnisse sind Tabelle 1 zu entnehmen:

Die Zeile "gemäß B1" enthält die Gardner-Farbwerte des erfindungsgemäßen Produktes gemäß Beispiel 1. Die Zeile "Standard" enthält die Gardner-Farbwerte eines handelsüblichen Cardanols (Cardanol von Firma Resibras).

**Tabelle 1**

| | sofort | 1 Tag | 2 Tage |
|---|---|---|---|
| gemäß B1 | 1,1 | 2,5 | 3,0 |
| Standard | 3 | 4,9 | 8 |

Es zeigt sich, dass das erfindungsgemäße Produkt dem handelsüblichen Standard bei weitem überlegen ist.

Darüber hinaus wurde festgestellt, dass Phenalkamine, die auf Basis des Produktes gemäß Beispiel 1, Aminen (insbesondre Diethyltetramin) und Formaldehyd hergestellt wurden, sich ebenfalls durch Farbstabilität auszeichneten.

## Patentansprüche

1. Verfahren zur Herstellung einer farbstabilem Zusammensetzung enthaltend Cardanol, wobei man in einem ersten Schritt rohes cashew nut shell liquid (CNSL) einer Destillation unterwirft, das dabei erhaltene Destillat in einem zweiten Schritt mit Essigsäureanhydrid umsetzt und das dabei erhaltene Reaktionsgemisch in einem dritten Schritt einer fraktionierenden Destillation unterwirft.

2. Verfahren gemäß Anspruch 1, wobei man in Schritt 1 eine Kurzwegdestillation durchführt.

3. Verfahren gemäß Anspruch 1 oder 2, wobei man in Schritt 2 Essigsäureanhydrid in einer Menge von 1 bis 5 Gew.-% - bezogen auf das aus Schritt 1 stammende Destillat - einsetzt.

4. Verfahren gemäß einem der Ansprüche 1 bis 3, wobei man die in Schritt 2 gebildete Essigsäure kontinuierlich aus dem System entfernt.

5. Verfahren gemäß einem der Ansprüche 1 bis 4, wobei man die Essigsäure nach Durchführung von Schritt 2 und vor Durchführung der fraktionierenden Destillation in Schritt 3 dadurch entfernt, dass man das in Schritt 2 gebildete Material einem Entgaser zuführt.

6. Verfahren gemäß einem der Ansprüche 1 bis 5, wobei man in Schritt 3 die Destillation in einer Fraktionierkolonne mit mehr als 6 theoretischen Böden durchführt.

7. Verfahren gemäß Anspruch 6, wobei die fraktionierende Destillation kontinuierlich durchführt.

8. Verfahren gemäß einem der Ansprüche 1 bis 7, wobei man das erhaltene Produkt zusätzlich einer Nachbehandlung durch Adsorbentien und/oder Reduktionsmittel unterzieht.

9. Verfahren gemäß Anspruch 8, wobei man im Anschluss an die Nachbehandlung eine Filtration durchführt.

## Claims

1. A process for the production of a color-stable composition containing cardanol, wherein, in a first step, crude cashew nut shell liquid (CNSL) is subjected to distillation, in a second step the distillate obtained is reacted with acetic anhydride and, in a third step, the reaction mixture obtained is subjected to fractional distillation.

2. A process as claimed in claim 1, wherein a short-path distillation is carried out in step 1.

3. A process as claimed in claim 1 or 2, wherein acetic anhydride is used in a quantity of 1 to 5% by weight, based on the distillate from step 1, in step 2.

4. A process as claimed in any of claims 1 to 3, wherein the acetic acid formed in step 2 is continuously removed from the system.

5. A process as claimed in any of claims 1 to 4, wherein, after step 2 and before the fractional distillation in step 3, the acetic acid is removed by feeding the material formed in step 2 to a degasifier.

6. A process as claimed in any of claims 1 to 5, wherein the distillation in step 3 is carried out in a fractionating column with more than 6 theoretical plates.

7. A process as claimed in claim 6, wherein the fractional distillation is carried out continuously.

8. A process as claimed in any of claims 1 to 7, wherein the product obtained is additionally aftertreated with adsorbents and/or reducing agents.

9. A process as claimed in claim 8, wherein the aftertreatment is followed by filtration.

## Revendications

1. Procédé pour la préparation d'une composition de couleur stable, contenant du cardanol, en soumettant, dans une première étape, du baume de cajou (cashew nut shell liquid - CNSL) brut à une distillation, en transformant le distillat obtenu dans une deuxième étape avec de l'anhydride de l'acide acétique et en soumettant le mélange réactionnel ainsi obtenu dans une troisième étape à une distillation fractionnée.

2. Procédé selon la revendication 1, en réalisant dans l'étape 1 une distillation instantanée.

3. Procédé selon la revendication 1 ou 2, en utilisant dans l'étape 2 de l'anhydride de l'acide acétique en une quantité de 1 à 5% en poids - par rapport au distillat provenant de l'étape 1.

4. Procédé selon l'une quelconque des revendications 1 à 3, en éliminant en continu du système l'acide acétique formé dans l'étape 2.

5. Procédé selon l'une quelconque des revendications 1 à 4, en éliminant l'acide acétique, après la réalisation de l'étape 2 et avant la réalisation de la distillation fractionnée dans l'étape 3, en ce qu'on introduit le matériau formé dans l'étape 2 dans un dégazeur.

6. Procédé selon l'une quelconque des revendications 1 à 5, en réalisant dans l'étape 3 la distillation dans une colonne de fractionnement présentant plus de 6 plateaux théoriques.

7. Procédé selon la revendication 6, la distillation fractionnée étant réalisée en continu.

8. Procédé selon l'une quelconque des revendications 1 à 7, le produit obtenu étant en plus soumis à un post-traitement par des adsorbants et/ou des réducteurs.

9. Procédé selon la revendication 8, une filtration étant réalisée après le post-traitement.
